# DEMANDE DE BREVET EUROPEEN

(11) **EP 1 302 170 A2**
(43) Date de publication de la demande: **16.04.2003**
(21) Numéro de dépôt: 02405870.3
(22) Date de dépôt: 10.10.2002
(51) Int. Cl.: A61B 17/86

(54) **Cheville ou vis pour coaptation pour petits os**

(30) Priorité: 16.10.2001 EP 01811008
(71) Demandeur: Inverlock Trading Limited, Nicosie (CY)
(72) Inventeur: Guintrand, Jacques, Nicosie (CY); Müller, Mireille, Nicosie (CY)
(74) Mandataire: Moinas, Michel

(57) **Abrégé**

La cheville ou vis médicale ou chirurgicale pour coaptation de *petits* os ou de fragments d'os comprend successivement, de son extrémité (3) vers sa tête (6), un premier filetage (2), puis un deuxième filetage (5) de pas différent dont la coopération lors du vissage dans la matière dure de l'os crée une force (f_{c}) tendant à rapprocher les deux os ou fragments d'os l'un de l'autre. La cheville ou vis (1) est constituée d'un matériau biorésorbable et sécable au bistouri électrique. Elle est pleine, c'est-à-dire non canulée, et ne présente pas de point ou de ligne de faiblesse.

La partie filetée (2) du coté de l'extrémité (3) est d'un diamètre inférieure à l'autre partie filetée (5).

Le matériau constitutif de la vis ou cheville est un acide polylactique ou un de ses dérivés ou une matrice chargée en acide polylactique ou un de ses dérivés.

## Description

La présente invention se rapporte à une cheville ou une vis médicale ou chirurgicale pour la coaptation de petits os ou de fragments d'os, étant entendu que par la désignation "os", on peut désigner aussi bien un os naturel qu'un os artificiel (une prothèse ou un fragment de prothèse).

Par l'expression "petits os", on entend désigner dans la suite de l'exposé les petits os proprement dits, ceux du pied ou de la main par exemple, ou les petits os qu'on peut rencontrer dans des structures ou articulations plus importantes, le coude ou l'épaule par exemple.

L'invention sera décrite pour une meilleure compréhension en relation avec la chirurgie du pied et pour des os naturels, mais on comprendra aisément, au vu de ce qui suit, qu'elle est applicable à n'importe quel type de chirurgie traumatique, réparatrice, correctrice ou esthétique, dès l'instant qu'il s'agît de joindre ou "recoller" deux os ou deux fragments d'os, naturels ou artificiels, par exemple réduire des fractures.

Dans la chirurgie du pied par exemple, les chirurgiens ont l'habitude d'avoir à leur disposition des boîtes de jeux de chevilles ou vis de différentes longueurs, sur une gamme s'étageant généralement de 12 à 32 mm. Ces jeux peuvent être composés de 12 à 32 vis de longueur différente, ou de même longueur. Dans ce cas, les vis présentent alors un point ou une ligne de faiblesse à des longueurs ou hauteurs différentes, de façon à permettre au chirurgien de sectionner là où il le faut la longueur excédentaire.

D'autre part, il convient également de disposer de chevilles ou vis qui soient bien sûr biocompatibles, mais de préférence aussi soient biorésorbables, c'est-à-dire dont le retrait n'est pas nécessaire après consolidation de l'os. Dans ce cas, l'os est venu coloniser la cheville qui a disparu petit-à-petit et s'est intégrée dans la matière osseuse par ostéosynthèse. Cela n'est évidemment pas possible avec des vis métalliques, en titane ou matériau plastique par exemple telles que décrites dans US 5'019'079, FR 2'787 989 ou WO 00/69352.

Le fait de pouvoir disposer d'une seule vis pour toute une gamme d'interventions, qui plus est de vis ou chevilles biorésorbables qui sont particulièrement onéreuses, conduirait à des simplifications en salle d'opération et à des économies substantielles.

En outre, les vis habituellement utilisées, pour des petits os au moins, sont canulées, c'est-à-dire qu'elles comportent un perçage longitudinal de part en part, dans lequel le chirurgien introduit une broche de guidage lors de la mise en place de la vis qui est auto-taraudante. La broche de guidage remplit aussi l'importante fonction de rigidifier l'ensemble et d'en augmenter la résistance mécanique, afin d'éviter les risques que la vis ne se brise, en particulier sur la ligne de faiblesse ou vers son extrémité.

A ce jour et à notre connaissance, personne n'a osé préconiser pour de petits os des vis pleines (non canulées) et en matière biodégradable, qui par nature ne présente pas la solidité du métal ou du plastique. Les vis décrites dans US 6'030'162, US 5'730 744 et WO 00/40165 sont par exemple toutes canulées.

Les chevilles sécables qu'on trouve sur le marché, souvent métalliques, ne sont habituellement pas sécables au bistouri électrique. De surcroît, elles ne sont sécables qu'au prix d'un point ou d'une ligne de faiblesse ménagée dans leur structure. Du coup, il faut prévoir à nouveau tout un jeu de chevilles de longueurs différentes jusqu'au point de scission ; on est donc ramené au problème évoqué précédemment, sans avoir résolu le problème de l'invention, celui de pouvoir disposer d'une cheville unique "universelle", passe-partout et multi-usages.

C'est précisément l'objet de l'invention, qui concerne une cheville ou vis (appelée indifféremment "cheville" ou "vis" dans la suite de l'exposé) médicale ou chirurgicale pour coaptation de deux os ou de deux fragments d'os, qui comprend successivement, de son extrémité vers sa tête, un premier filetage, puis un deuxième filetage de pas différent dont la coopération lors du vissage dans la matière dure de l'os crée une force tendant à rapprocher les deux os ou fragments d'os l'un de l'autre. Cette cheville ou vis est pleine et ne présente pas de point ou de ligne de faiblesse. Elle est constituée d'un matériau biorésorbable et sécable au bistouri électrique.

Les deux parties filetées de la cheville sont séparées ou non par une partie non filetée et avantageusement aussi la partie filetée du coté de l'extrémité est d'un diamètre inférieure à l'autre partie filetée. Ainsi décrite, la vis est à double pas et à double diamètre.

La cheville peut être réalisée en tout matériau biocompatible et biorésorbable, par exemple en acide polylactique ou un de ses dérivés, qui est facilement sécable au bistouri électrique, c'est-à-dire sous l'action de l'arc électrique généré par le bistouri. En variante, on peut utiliser comme matériau une matrice chargée en acide polylactique ou un de ses dérivés. Tout matériau de type acide polylactique PLA ou PLLA est utilisable, par exemple un matériau composé de 70% d'acide L-polylactique et de 30% d'acide DL-polylactique, qui se résorbe lentement.

L'invention sera mieux comprise en référence aux dessins annexés, donnés à titre d'exemple non limitatifs. Sur ces dessins, il faut bien comprendre que la vis est non canulée, c'est-à-dire pleine.

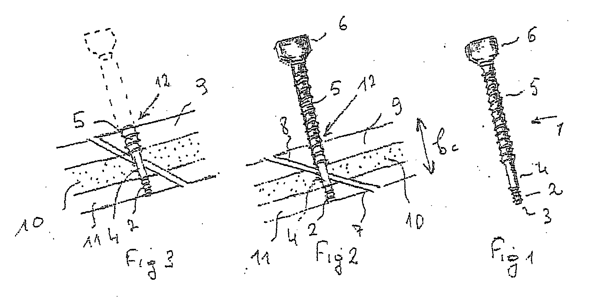

La structure de la vis ou cheville se lit 1 de façon très simplement sur la figure 1. A son extrémité, on trouve une première partie filetée 2, dont le filetage vient avantageusement en bout 3 comme représenté sur la figure. La première partie filetée 2 suivie d'une partie lisse 4, puis d'une deuxième partie filetée 5, d'un pas légèrement différent, de façon que lors du vissage, à l'ancrage dans les deux parties osseuses, les deux parties subissent une force de compression (f_{c}) tendant à les rapprocher. Après la deuxième partie filetée 5, on trouve directement, comme représenté sur la figure, ou en variante non représentée par l'intermédiaire d'autre autre partie lisse, la tête 6 de vis.

Lorsque le chirurgien veut réaliser une ostéotomie, il aboute les deux faces 7, 8 dans lesquelles il fore un perçage de part en part de diamètres correspondants à ceux de la vis qu'il souhaite mettre en place. Pour la chirurgie du pied, il utilisera par exemple un vis d'un diamètre de 3,5 mm en partie proximale pour un diamètre de 2,5 ou 2,6 mm en partie distale, c'est-à-dire en extrémité.

En l'occurrence, comme cela découle de la figure 2, le chirurgien réalise un premier perçage à travers les faces 7,8. Il perce d'abord dans la paroi corticale osseuse 9, puis le partie spongieuse 10 de l'os et enfin la deuxième paroi corticale osseuse 11 (en position inférieure sur la figure). Ce premier perçage est au plus petit des diamètres, celui de l'extrémité de la cheville où se trouve le premier filetage 2. Pour ce faire, il utilise de préférence une mèche taraudante.

Puis, changeant d'outil, le chirurgien agrandit le diamètre du premier perçage au diamètre du deuxième filetage 5, qui sur la figure est aussi celui de la partie lisse 4, en veillant à ne pas à ne pas dépasser, au plus, la partie spongieuse 10 pour ne pas entamer la deuxième paroi osseuse 11. Pour ce faire, il utilise de préférence une mèche taraudante épaulée, c'est-à-dire une mèche munie d'un arrêt ou d'une butée, réglée préalablement à la bonne longueur

En variante non représentée, les deux filetages sont placés à la suite l'un de l'autre, en sorte que la partie lisse 4 est inexistante.

Le chirurgien présente la cheville à l'entrée du perçage et la visse. La cheville vient se visser par son extrémité avec le premier filetage 2 dans la deuxième paroi 11 de l'os, tout en se vissant simultanément à l'aide de son deuxième filetage 5 dans la première paroi 9 de l'os en exerçant une force de compression.

Cette force de compression (f_{c}) résulte du fait que deux filetages 3,5 ont des pas légèrement différents, le pas du premier filetage 3 étant plus petit que le pas du deuxième filetage 5, ce qui induit une force de rapprochement en les deux parois corticales de l'os ou des os.

Il reste alors au chirurgien à couper la cheville à ras de l'os, en position 12, pour qu'elle ne fasse pas protubérance, ce qu'il réalise au bistouri électrique.

Le chirurgien a pu faire tout ce travail sans utiliser de broche de guidage, puisque la vis n'est pas canulée. Contre toute attente, l'expérience a montré que les risques de brisure de la vis à un quelconque endroit, sont insignifiants, voire inexistants, malgré les efforts importants exercés sur elle.

La cheville, intra-osseuse, va rester en place à demeure et ne nécessite pas d'être enlevée. Etant biorésorbable, elle va petit-à-petit s'assimiler dans l'os par ostéosynthèse, ce qui pour un dérivé polylactique est réalisé en un temps de l'ordre de un an.

On aura compris que de la sorte un seul type de cheville peut être utilisé et que sa longueur n'a pas d'importance, dans la mesure où le chirurgien, en la sectionnant au bistouri électrique, l'ajuste très exactement à l'épaisseur des os sur lesquels il travaille. De sérieuses économies sont alors réalisées, puisqu'il n'est pas nécessaire de tenir à disposition toute une gamme de longueur de chevilles dont certaines d'ailleurs ne seraient utilisées que rarement.

## Revendications

1. Cheville ou vis médicale ou chirurgicale pour coaptation de petits os ou de fragments d'os, comprenant successivement, de son extrémité (3) vers sa tête (6), un premier filetage (2), puis un deuxième filetage (5) de pas différent dont la coopération lors du vissage dans la matière dure de l'os crée une force (f_{c}) tendant à rapprocher les deux os ou fragments d'os l'un de l'autre, **caractérisée en ce que** la cheville ou vis (1) est pleine, ne présente pas de point ou de ligne de faiblesse et est constituée d'un matériau biorésorbable et sécable au bistouri électrique.

2. Cheville ou vis selon la revendication 1, **caractérisée en ce que** les deux parties filetées (2,5) sont séparées ou non par une partie non filetée (4).

3. Cheville ou vis selon la revendication 1 ou 2, **caractérisée en ce que** la partie filetée (2) du coté de l'extrémité (3) est d'un diamètre inférieure à l'autre partie filetée (5).

4. Cheville ou vis selon l'une des revendications précédentes, **caractérisée en ce que** le matériau de la vis ou cheville (1) est un acide polylactique ou un de ses dérivés ou une matrice chargée en acide polylactique ou un de ses dérivés.
